(19) Europäisches Patentamt

European Patent Office

Office européen des brevets

(11) Veröffentlichungsnummer: **0 321 755 B1**

(12) **EUROPÄISCHE PATENTSCHRIFT**

(45) Veröffentlichungstag der Patentschrift: **22.02.95**

(51) Int. Cl.⁶: **C08J 3/12**, A61L 15/00

(21) Anmeldenummer: **88120064.6**

(22) Anmeldetag: **01.12.88**

Verbunden mit 89900220.8/0389547
(europäische
Anmeldenummer/Veröffentlichungsnummer)
durch Entscheidung vom 11.02.92.

(54) **Verfahren zur Agglomerierung wasserquellbarer Polymere durch Sintergranulation.**

(30) Priorität: **04.12.87 DE 3741157**

(43) Veröffentlichungstag der Anmeldung:
**28.06.89 Patentblatt 89/26**

(45) Bekanntmachung des Hinweises auf die
Patenterteilung:
**22.02.95 Patentblatt 95/08**

(84) Benannte Vertragsstaaten:
**AT BE CH DE ES FR GB GR IT LI LU NL SE**

(56) Entgegenhaltungen:
**EP-A- 0 001 706**
**GB-A- 1 376 091**
**GB-A- 2 040 954**

(73) Patentinhaber: **Chemische Fabrik Stockhausen GmbH**
**Bäkerpfad 25**
**D-47805 Krefeld (DE)**

(72) Erfinder: **Brehm, Helmut**
**Dachsstrasse 22**
**D-4150 Krefeld (DE)**

(74) Vertreter: **Klöpsch, Gerald, Dr.-Ing. Patentanwalt**
**An Gross St. Martin 6**
**D-50667 Köln (DE)**

## Beschreibung

Die Erfindung betrifft Granulate wasserquellbarer Polymerer, Verfahren zu ihrer Herstellung und ihre Verwendung zur Absorption von wässrigen Lösungen, insbesondere Körperflüssigkeiten wie Blut und/oder Urin.

Wasserquellbare Polymere werden heute vielfältig, insbesondere im Bereich der Hygiene zur Aufnahme von Körperflüssigkeiten wie z.B. Blut und/oder Urin verwendet. Hierbei müssen die wasserquellbaren Polymeren in der Lage sein, große Mengen Flüssigkeit (Wasser oder Körperflüssigkeiten) in sehr kurzer Zeit zu binden. Diese bekannten wasserquellbaren feinteiligen Polymeren weisen jedoch den Nachteil auf, zum "Blocken" zu neigen, d.h. beim Kontakt mit Wasser oder wässrigen Lösungen verklebt die äußere Schicht des Haufwerks und verhindert so das weitere Vordringen der Flüssigkeit in das Innere des Absorptionsmittels.

Die wasserquellbaren pulverförmigen feinteiligen Polymeren fallen je nach Herstellungweise in einem mehr oder weniger breiten Kornspektrum an, das von Feinstaub über gröberes Korn bis zu Grobkorn reicht. Ein typisches Kornspektrum für ein nach der Trocknung vermahlenes wasserquellbares Polymer liegt im Bereich von 10 bis 800 $\mu$m, wobei die Kornfraktion von 150 bis 63O $\mu$m für praktische Zwecke als Absorptionsmaterial eingesetzt wird. Der anfallende Feinstaub einer Korngröße von etwa 10 bis 150 $\mu$m ist zu fein, um als Absorptionsmaterial eingesetzt zu werden, ist insoweit als Abfall anzusehen und stellt darüberhinaus eine Belästigung dar.

Aufgabe der Erfindung ist es daher, derartige wasserquellbare, pulverförmige Polymere in einer neuen Erscheinungsform bereitzustellen, die den Nachteil des Blockens nicht aufweist, rasch große Flüssigkeitsmengen aufnehmen kann und die außerdem in einem einfachen und wirtschaftlichen Verfahren hergestellt werden kann.

Diese Aufgabe wird erfindungsgemäß gelöst durch ein Granulat des wasserquellbaren Polymeren, das erhältlich ist durch Agglomerierung mit Hilfssubstanzen im folgenden auch Agglomerierungshilfsmittel genannt, die aufgrund ihrer Schmelz- bzw. Erweichbarkeit zur Agglomerierung der feinteiligen Polymerpartikel im Stande sind.

Die Kornvergrößerung durch Agglomeration feindisperser Materialien ist an sich bekannt. Zum Aufbau von Granulaten aus Pulverpartikeln stehen verschiedene Verfahren zur Verfügung (Ullmann's Enzyklopädie der techn. Chemie, 4.Auflage Bd. 18, S. 157; Chem.Ind. 53 (1981) S. 37-41), darunter auch die Sinter- oder Schmelzagglomerierung. Die Aufbaugranulierung pulverförmiger, wasserquellbarer Polymerisate hat bisher jedoch keinen Eingang in die Technik gefunden.

Überraschenderweise hat sich gezeigt, dass pulverförmige, wasserquellbare Polymere durch Erhitzen von Mischungen mit pulverförmigen, schmelzbaren, thermo- und duroplastischen Substanzen als Agglomerierungshilfsmittel agglomeriert werden können, wobei die Stabilität der Agglomerate erhöht und hierdurch ihre Verarbeitung und ihre Verwendung deutlich verbessert wird. Es ist ferner überraschend, dass die so hergestellten Granulate praktisch keine Neigung zum "Blocken" haben, sondern vielmehr in sehr kurzer Zeit große Flüssigkeitsmengen aufnehmen können.

Diese überraschende Verbesserung ihrer Eigenschaften zeigen alle agglomerierten, wasserquellbaren Polymeren wie z.B. vernetzte Homo-, Co- und Terpolymerisate der Acryl- und Methacrylsäure, Pfropfpolymere von Acrylderivaten auf Stärke- und Celluloseprodukte, Polyurethane, Verseifungsprodukte von vernetztem Homo- und Copolymeren des (Meth-)acrylnitrils, der (Meth-)acrylamide und (Meth)Acrylester sowie Copolymere aus Isobutylen und Maleinsäureanhydrid. Die Polymerisate können weitere ionogene und nichtionische Monomere enthalten, wie z.B.: Acrylamidopropansulfonsäure, Vinylphosphonsäure, Vinylsulfonsäure, Dialkylaminoalkyl(meth-)acrylate, Dialkylaminoalkyl(meth-)acrylamide, Quaternierungsprodukte tert. Aminogruppen tragender Monomere, ein oder mehrere Allylgruppen enthaltende Monomere, (Meth-)-Acrylamid, Alkyl(Meth-)acrylamide, Methylenbisacrylamid, Mono-, Di- und Triacrylsäurealkylester, Hydroxialkyl(meth-)acrylester sowie Epoxidgruppen tragende Verbindungen. Die wasserquellbaren Polymeren können ganz oder teilweise als Ammonium-, Alkali- oder Erdalkalisalze, allein oder in Mischung vorliegen.

Insbesondere werden als wasserquellbaren Polymere Polymerisate von (Meth-)acrylsäure, Polymerisate von wenigstens teilweise verseiftem (Meth-)acrylnitril, Polymerisate von wenigstens teilweise verseiftem (Meth-)acrylamid, Polymerisate von wenigstens teilweise verseiften (Meth-)acrylsäureestern, Pfropfmischpolymere von (Meth-)acrylnitril oder (Meth-)acrylamid mit Stärke oder Cellulose oder deren Derivaten, Polyurethane und/oder Polymerisate aus Isobutylen und Maleinsäureanhydrid bevorzugt.

Die Herstellung dieser Polymerisate ist bekannt. Sie kann nach verschiedenen Verfahren erfolgen, wie z.B.: Lösungs-, Fällungs-, Suspensions-, Perl- oder Pfropfpolymerisation.

Als Agglomerierungshilfsmittel könnnen alle pulverförmigen, synthetischen oder natürlichen schmelzbaren thermo- bzw. duroplastischen Stoffe allein oder in Mischung verwendet werden, die einen Schmelz-/Erweichung-/punkt/bereich haben. Beispielhaft, aber nicht begrenzend seien genannt:

Als synthetische Agglomerierungshilfsmittel sind geeignet: Polyolefine und ihre Co- und Terpolymeren, Poly- und Copolyamide, Poly- und Copolyester, Poly(meth-)acryl-ate, -nitrile und ihre Co- und Terpolymeren, Polyvinyl- und Allylester und -ether, ihre Copolymerisate und ihre Verseifungsprodukte sowie deren Acetale, Polyalkylenglykole, Polyalkylenoxide sowie polyalkoxilierte Verbindungen, PVC und seine Co- und Terpolymeren (z.B.mit Vinylacetat oder Maleinsäureestern) sowie seine Mischungen mit anderen Polymeren, Polystyrol, Styrol-Acrylnitril und Acrylnitril-Butadien-Styrol-Polymerisate einschließlich ihrer Mischungen. Polycarbonate und Polyurethane sind in Pulverform Ebenfalls geeignet. Verwendbar sind auch Epoxidharze, Aminoplaste, Phenoplaste allein oder in Verbindung mit einem Härter.

Auch natürliche Stoffe, ihre Derivate und Umwandlungsprodukte sind hervorragende Agglomerationspulver, wie z.B. Celluloseester, Schellack, Kolophonium und Kolophoniumester sowie hydriertes und disproportioniertes Kolophonium, sowie natürliche Thermoplaste auf Basis Hydroxibuttersäure und -valeriansäure. Weiter zu nennen sei die Gruppe der Fettsäuren und Fettsäurederivate wie z.B. die Fettsäureester, -amide, -amine sowie Metallseifen.

Als zusätzliche, nicht agglomerierend wirkende Bestandteile können Trennmittel wie pyrogenes $SiO_2$ - (Aerosil [R])oder $Al_2O_3$ oder Calciumsilicat ("Silcasil") in Mengen von 0,1 - 1 Gew.-% der zu agglomerienden Pulvermischung zugesetzt werden.

Die Agglomeration erfolgt nach der kontinuierlichen oder diskontinuierlichen Vermischung des pulverförmigen, wasserquellbaren Polymeren mit dem pulverförmigen Agglomerierungshilfsmittel durch Erwärmen der Mischung. Die Erwärmung wird in bewegter oder ruhender Schicht ausgeführt. Für die Erwärmung der Pulvermischung stehen verschiedene Verfahren zur Verfügung, z.B. durch Kontakt mit beheizten Flächen oder Heißgasen, durch Strahlungswärme oder Hochfrequenzerwärmung. Als geeignete, beheizbare Apparate sein genannt: Freifall- und Zwangsmischer, Wärmeschränke und Drehrohröfen, Bänder und Vibrationsrinnen sowie das Wirbelbett. Die erforderliche Agglomerationstemperatur ist abhängig vom Agglomerierungshilfsmittel und reicht theoretisch von Temperaturen weit unter 0°C bis zur Zersetzungstemperatur der wasserquellbaren Polymeren. Entscheidend ist, dass das Agglomerierungshilfsmittel und wasserquellbares Polymerisat bei Temperaturen miteinander gemischt werden, bei denen beide Stoffe pulverförmig vorliegen und anschließend durch Erwärmen der Pulvermischung die Agglomeration erfolgt. Die Agglomerationszeit ist abhängig davon, wie schnell die Pulvermischung erwärmt werden kann, also vom Wärmeübergang und der Temperaturdifferenz. Zeiten zwischen 1 Minute und 1 Stunde sind - abhängig vom Verfahren, Temperatur und Schichtstärke - erforderlich.

Der Verlauf der Agglomeration kann in einem beheizbaren, transparenten Freifallmischer leicht optisch verfolgt werden.

Die zur Agglomerierung eingesetzten Hilfssubstanzen sind im Bereich von -100 bis +300°C, bevorzugt im Bereich von 20 bis 200°C und insbesondere im Bereich von 50 bis 160°C pulverförmig und erweichen bzw. schmelzen oberhalb dieser Temperaturen. Es sind also auch solche Hilfssubstanzen geeignet, die erst bei tieferen Temperaturen, z.B. -100°C pulverförmig sind, dagegen beim Raumtemperatur z.B. klebrig. Solche Substanzen werden durch Kaltmahlung bei tiefen Temperaturen, bei denen sie spröde sind, pulverisiert, kalt mit den Polymeren vermischt und anschließend z.B. auf Raumtemperatur erwärmt.

Die Menge an pulverförmigem Agglomerierungshilfsmittel hängt ab von seiner Art, Korngröße und dem Verwendungszweck des Granulates. Die Granulate können 75 bis 99,5, vorzugsweise 80 bis 98 Gew.-% wasserquellbares Polymeres bzw. 25,5 bis 0,5, vorzugsweise 20 bis 2 Gew.-% pulverförmiges Agglomerierungshilfsmittel enthalten. Bevorzugt wird als Ausgangsmaterial ein wasserquellbares Polymer einer Teilchengröße von 10 bis 150, vorzugsweise 10 bis 100μm eingesetzt.

Die zur Agglomerierung verwendeten Hilfssubstanzen können zusätzlich nicht agglomerierend wirkende Verbindungen wie Füllstoffe, Weichmacher, Fliessverbesserer, Trennmittel, Härter, Antistatika, Stabilisatoren und/oder Schäumer enthalten.

Nach der Agglomeration wird das Granulat gesieht und die gewünschte Kornfraktion, die einen Vergleich mit einem nicht granulierten, wasserquellbaren Polymeren gleicher Korngröße zuläßt, hinsichtlich seiner Aufnahmegeschwindigkeit in Kochsalzlösung geprüft. Das Überkorn wird zerkleinert und das Unterkorn zurückgeführt.

Die Erfindung betrifft ferner Wegwerferzeugnisse für Hygieneartikel, wie z.B. Windeln oder Damenbinden, enthaltend als Absorptionsmittel für Körperflüssigkeiten, wie Wasser und Urin ein Granulate nach Anspruch 1.

Prüfung:

Gemessen wird die aufgenommene Menge an Flüssigkeit pro Gramm wasserquellbares Polymeres (granuliertes Polymeres).

Eine gewogene Menge Polymeres wird in einen Teebeutel eingeschlossen und in eine 0,9%ige Kochsalzlösung getaucht. Ermittelt wird die Aufnahmemenge nach

a) 15 Sekunden und

b) 5 Minuten mit nachfolgendem Abschleudern bei 1400 Upm,

wobei die Menge Flüssigkeit, die das Material des Teebeutels aufgenommen hat, abgezogen wird.

$$\text{Aufnahme-menge} \left[\frac{g}{g}\right] = \frac{\text{Gewicht nach der Adsorption } [g] - \text{Gewicht des Teebeutels } [g]}{\text{Gewicht des Polymeren } [g]}$$

Es zeigt sich, dass die Agglomerate sowohl einem nichtagglomerierten wasserquellbaren pulverförmigen Polymeren vergleichbarer Korngröße als auch dem als Ausgangsmaterial der Agglomerierung verwendeten Feinkorn insbesondere in der Geschwindigkeit der Flüssigkeitsaufnahme deutlich überlegen sind.

Vergleichspolymere

Vergleichspolymerisat (handelsüblich)

| Art: | Vernetztes Lösungspolymerisat auf Basis Acrylsäure, das zu 70 Mol% als Na-Salz vorliegt. |
|---|---|
| Kornfraktion: | 150 - 630 μm |
| Aufnahme nach 15 Sek.: | 12 g/g |
| Aufnahme nach 5 Min.: | 39 g/g |

Beispiel 1

95 g Vergleichspolymerisat der Korngröße kleiner als 90 μm werden mit 5 g Co-Polyester der Korngröße 60 bis 200 μm, einem Schmelzbereich von 115 - 125°C, einem Schmelzindex von 34 g/10 Min. [1] und einer Schmelzviskosität von 3600 dPas bei 160°C in einem Intensivmischer 5 Minuten gemischt. Zur Agglomeration überträgt man das Pulvergemisch in einen beheizbaren Freifallmischer aus Glas. Bei einer Manteltemperatur von 160°C beginnt nach 2 Min. deutlich sichtbar die Agglomeration. Nach weiteren 5 Min. wird das Granulat entnommen und im Luftstromklassierer in die Kornfraktion < 150μm, 150 - 630 μm und > 630μm aufgeteilt. Die Hauptfraktion 150 - 630 μm wird geprüft:

| Ausbeute an Granulat: | 85% |
|---|---|
| Aufnahme nach 15 Sek.: | 37 g/g |
| Aufnahme nach 5 Min.: | 41,8 g/g |

Beispiel 2 - 6

Verfahren wird wie in Beispiel 1. Zur Agglomeration verwendet wird das Vergleichs-polymerisat der Korngröße < 90 μm.

| Bei-spiel | Agglomeration Hilfsmittel | | | | | Bedingungen | | Prüfung Granulat 150-630 μm | |
|---|---|---|---|---|---|---|---|---|---|
| | Menge % | Bezeichnung | Korn-gröpe μm | Schmelz-bereich °C | -index g/10 Min. | Zeit Min. | Temp. °C | Aufnahme n.15 Sek. g/g | Aufnahme n.5 Min. g/g |
| 2 | 5 | Copolyamid[4] | 0-80 | 115-125 | 15[1] | 8 | 160 | 28,8 | 41,3 |
| 3 | 5 | HD-Polyethylen | 1-300 | 100-105 | 200[2] | 5 | 160 | 35,9 | 36,4 |
| 4 | 5 | Ethylen-Vinylace-tat Copolym. | 1-300 | 70-80 | 160[1] | 10 | 160 | 23,2 | 37,2 |
| 5 | 5 | HD-Polyethylen | 5-74 | 109-112 | 7[3] | 10 | 160 | 45,0 | 41,5 |
| 6 | 5 | Co-Polyamid[5] | 0-80 | 80-90 | 65[1] | 8 | 160 | 36,1 | 42,1 |

[1] MFI 160°C / 2,16
[2] MFI 190°C / 2,16
[3] MFI 190°C / 2
[4] R Platamid H 005, Deutsche Atochem, Bonn
[5] "       H 103,  "       ,       "

Beispiel 7 - 15

Vergleichspolymerisat der Korngröße kleiner als 90 μm wird mit dem pulverförmigen ; Agglomerie-rungshilfsmittel I gemischt und die Pulvermischung in Trockenschrank für 30 Min. in einer Schichtstärke von 4 cm gelagert. Nach dem Abkühlen wird der Pulverkuchen zerdrückt und gesiebt.

| Beispiel | Agglomeration | | | |
|---|---|---|---|---|
| | Hilfsmittel | | Temp. | Granulat 150 – 630 µm Aufnahme nach 15 sec. |
| | Menge % | Bezeichnung | °C | g/g |
| 7 | 5 | 90-EO-Stearinsäure | 160 | 36,1 |
| 8 | 5 | Polyethylenoxid Molgew.ca. $4\times10^6$ | 225 | 37,8 |
| 9 | 5 | Calciumlaurat | 160 | 27,6 |
| 10 | 5 | Hochdr.-Polyethylen | 160 | 35,9 |
| 11 | 2 | Co-Polyamid[1] Schmelzbereich 110-120°C Korngröße 0-80µm | 160 | 23,6 |
| 12 | 5 | " " | 130 | 33,5 |
| 13 | 5 | Co-Polyamid[2] Schmelzbereich 115-130°C Korngröße 0-80µm | 160 | 42,5 |

| 14 | 5 | Terpolymerisat aus Acrylsäure, Ethylen, Acrylsäureester | 130 | 29,5 |
| | | Korngröße 5-74 μm | | |
| | | MFI 190/2: 7g/10 min | | |
| | | Dichte: 0,929g/cm$^3$ | | |
| | | Schmelzber.:98-102°C | | |
| 15 | 5 | Ethylen-Vinylacetat-Copolymerisat | 160 | 31,5 |
| | | Korngröße 5-74 μm | | |
| | | MFI 190/2: 4g/10min | | |
| | | Schmelzbereich: 92-95°C | | |

1)® Griltex 1, Emser Werke AG

2)® Platamid M 840 PA, Deutsche Atochem, Bonn

Beispiel 16

Ein beheizbarer Schneckenmischer wird mit 14,25 kg Vergleichspolymerisat der Korngröße kleiner als 90 μm und 0,75 kg pulverisierter Citronensäure-Monohydrat beschickt. Nach einer Mischzeit bei Raumtemperatur von 10 Min. wird die Manteltemperatur durch Beheizen mit Dampf auf 160°C erhöht. Nach 8 Min. wird das Granulat ausgeschleust, abgekühlt und gesiebt.

Die Kornfraktion 150 - 630 μm zeigt eine Aufnahmegeschwindigkeit für 0,9%ige Kochsalzlösung von 26 g/g in 15 Sekunden.

Beispiel 17

In einem haushaltsüblichen Microwellenherd mit einem Anschlußwert von 1400 Watt wird eine Pulvermischung aus 180 g Vergleichspolymerisat der Korngröße kleiner als 150 μm und 20 g Co-Polyamidpulver (wie in Beispiel 6) für 3 Min. gelagert. Die Endtemperatur beträgt 95°C. Nach dem Abkühlen wird der Pulverkuchen zerdrückt und gesiebt.

Die Kornfraktion 150 - 630 μm zeigt eine Aufnahmegeschwindigkeit für 0,9%ige Kochsalzlösung von 28,5 g pro Gramm Granulat in 15 Sek.

Beispiel 18

In ein Wirbelbett werden 1,54 kg der Pulvermischung wie in Beispiel 15 eingetragen. Die Schichthöhe beträgt ruhend 100 mm. Bei einer Luftgeschwindigkeit von 0,33 m/s und einer Zulufttemperatur von 180°C erfolgt die Granulation nach 28 Min. bei einer Produkttemperatur von 130°C. Die Granulatausbeute der Kornfraktion 150 - 630 μm beträgt 86,5 Gew.%. Dieses Granulat zeigt eine Aufnahmegeschwindigkeit für 0,9%ige Kochsalzlösung von 33,5 g pro Gramm Polymeres in 15 Sek.

Beispiel 19 - 21

Vergleichspolymerisat der Korngröße kleiner als 150 μm wird mit dem pulverförmigen Agglomerierungshilfsmittel gemischt und im Wirbelbett entsprechend Beispiel 18 behandelt.

| Beispiel | Agglomeration Hilfsmittel | | | Prüfung Granulat 150-630 μm Aufnahme nach 15 Sek (g/g) |
|---|---|---|---|---|
| | Menge (%) | Bezeichnung | Temp. (°C) | |
| 19 | 5 | Polyurethan Schmelzbereich:160-180°C Korngröße: 0-500 μm Shore-A-Härte: 82±2 nach DIN 53505 | 140 | 29,3 |
| 20 | 5 | Polystyrol MFI 200/5:25g/10 Min. Korngröße: 5-90 μm | 160 | 25,0 |
| 21 | 5 | Niederdruck Polyethylen MFI 190/2:10g/10 Min. Korngröße: 5-90 μm Schmelzbereich: 131-134 | 160 | 30,2 |

Beispiel 22:

Eine Pulvermischung aus 1455 g Vergleichspolymerisat der Korngröße < 100 μm, 45 g Hochdruckpolyethylen der Korngröße 5 - 74 μm, der Dichte 0,924 g/cm$^3$ und dem Schmelzindex 7 g/10 Min (MI 190/2) sowie 1,5 g Aerosil® 200 [3] werden in einem Wirbelbett bis zur Produkttemperatur 140°C behandelt. Die Granulatausbeute beträgt:

16,3 % der Korngröße > 630 μm

82,2 % der Korngröße 150 - 630 μm

1,5 % der Korngröße < 150 μm

Die Kornfraktion 150 - 630 μm zeigt eine Aufnahmegeschwindigkeit von 25,4 g 0,9 %ige Kochsalzlösung pro Gramm Granulat in 15 Sekunden.

Die folgende Prüfung soll die überraschende Festigkeit der Sintergranulate im gequollenen Zustand demonstrieren. Diese Festigkeit des gequollenen Gels hat eine große Bedeutung für die Verwendung von wasserquellbaren Polymeren z.B. in Windeln. Je größer die Festigkeit des Gels, desto geringer ist die Gefahr des Austretens aus der Windelkonstruktion.

Zur Prüfung wird die Kornfraktion 200 - 300 μm, dem Demand absorbency test, beschrieben in: Allgemeiner Vliesstoff-Report 5/87, S. 210- 218, unterworfen und das gequollene Gel nach Erreichen der max. Aufnahme auf Festigkeit geprüft.

5 g max. gequollenes Gel gibt man in eine 10 ml Kolbenpipette, an deren Boden ein 300-μm-Sieb fixiert ist. Unter steigender Gewichtsbelastung des Kolbens wird der Druck bestimmt, zu dem Gel durch die Sieböffnungen gedrückt wird. Die Last auf dem Kolben der Pipette wird in g pro cm$^2$ angegeben.

| Produkt n.Beispiel Nr. | max. aufgenommene Flüssigkeitsmenge ml/g | Belastung g/cm$^2$ |
|---|---|---|
| Vergleichspolymerisat | 57 | 700 |
| 5 | 56 | 900 |
| 14 | 55,1 | 1060 |
| 15 | 57 | 760 |

## Patentansprüche

1. Granulate von wasserquellbaren Polymeren, gekennzeichnet durch einen Gehalt an 75 bis 99,5, vorzugsweise 80 bis 98 Gew.-% wasserquellbarem Polymer und 25 bis 0,5, vorzugsweise 20 bis 2 Gew.-% an einem pulverförmigen Agglomerierungshilfsmittel, welches die Agglomerierung allein durch Temperaturerhöhung und dadurch ausgelöst des Schmelzen bzw. Erweichen bewirkt, sowie gegebenenfalls weiteren zusätzlichen, nicht agglomerierend wirkenden Bestandteilen, erhältlich durch Erwärmen eines wasserquellbaren pulverförmigen, fein dispersen Polymeren einer Teilchengröße von 10 bis 150, vorzugsweise 10 bis 100 μm, mit dem pulverförmigen Agglomerierungshilfmittel in Abwesenheit

[3] Fa. Degussa

8

von freiem Wasser.

2. Verfahren zur Herstellung von Granulaten wasserquellbarer Polymerer gemäß Anspruch 1 durch Sinter-Agglomeration von pulverförmigen, fein dispersen Ausgangsmaterialien, dadurch gekennzeichnet, daß man eine Mischung, die 75 bis 99,5, vorzugsweise 80 bis 98 Gew.-% des pulverförmigen, wasserquellbaren Polymeren und 25 bis 0,5, vorzugsweise 20 bis 2 Gew.-% eines pulverförmigen Agglomerierungshilfsmittels, das aufgrund seiner Schmelz- bzw. Erweichbarkeit durch Temperaturerhöhung agglomerierend wirkt, enthält, in Abwesenheit von freiem Wasser erwärmt.

3. Verfahren nach Anspruch 2, dadurch gekennzeichnet, daß man als Ausgangsmaterial ein wasserquellbares Polymer einer Teilchengröße von 10 bis 150, vorzugsweise 10 bis 100 μm einsetzt.

4. Verfahren nach einem der Ansprüche 2 bis 3, dadurch gekennzeichnet, daß man eine Mischung erhitzt, die 75 bis 99,5, vorzugsweise 80 bis 98 Gew.-% wasserquellbares Polymer und 25 bis 0,5, vorzugsweise 20 bis 2 Gew.-% Agglomerierungshilfsmittel enthält.

5. Verfahren nach einem der Ansprüche 2 bis 4, dadurch gekennzeichnet, daß man als Agglomerierungshilfsmittel Substanzen verwendet, die im Bereich von -100 bis +300°C, bevorzugt im Bereich von 20 bis 200 °C und insbesondere im Bereich von 50 bis 160 °C pulverförmig sind.

6. Verfahren nach einem der Ansprüche 2 bis 5, dadurch gekennzeichnet, daß man als Agglomerierungshilfsmittel synthetische oder natürliche Stoffe allein oder in Mischung untereinander verwendet, die einen Schmelz-/Erweichungs-/punkt/bereich aufweisen.

7. Verfahren nach Anspruch 8, dadurch gekennzeichnet, daß man als Agglomerierungshilfsmittel Thermoplaste und/oder Duroplaste verwendet.

8. Verfahren nach einem der Ansprüche 2 bis 7, dadurch gekennzeichnet, daß man als synthetische Agglomerierungshilfsmittel Polymerisate auf der Basis von Polyolefinen, Polyamiden, Polyestern, Poly-(meth-)acrylaten, Poly(meth-)acrylnitrilen, Polyalkylenoxiden, Polyvinylchlorid, Polystyrol, Polycarbonaten und/oder Polyurethanen einsetzt.

9. Verfahren nach einem der Ansprüche 2 bis 6, dadurch gekennzeichnet, daß man als synthetische Agglomerierungshilfsmittel Epoxiharze, Aminoplaste, Phenoplaste allein oder zusammen mit einem Härter verwendet.

10. Verfahren nach einem der Ansprüche 2 bis 6, dadurch gekennzeichnet, daß man als natürliche Agglomerierungshilfsmittel Celluloseester, Schellack, Kollophonium und dessen Derivate oder natürliche Thermoplaste auf der Basis von Fettsäuren und Fettsäurederivaten einsetzt.

11. Verfahren nach einem der Ansprüche 2-10, dadurch gekennzeichnet, daß die Agglomerierungshifsmittel zusätzlich nichtagglomerierend wirkende Verbindungen wie Füllstoffe, Weichmacher, Fließverbesserer, Trennmittel, Härter, Antistatika, Stabilisatoren und/oder Schäumer enthalten.

12. Verfahren nach einemd er Ansprüche 2 bis 11, dadurch gekennzeichnet, daß man als wasserquellbare Polymere Polymerisate von (Meth-)acrylsäure, Polymerisate von wenigstens teilweise verseiftem (Meth-)acrylnitril, Polymerisate von wenigstens teilweise verseiftem (Meth-)acrylamid, Polymerisate von wenigstens teilweise verseiften (Meth-)acrylsäureestern, Pfropfmischpolymere von (Meth-)acrylnitril oder (Meth-)-acrylamid mit Stärke oder Cellulose oder deren Derivaten, Polyurethane und/oder Polymerisate aus Isobutylen und Maleinsäureanhydrid verwendet.

13. Verfahren nach einem der Ansprüche 2 bis 12, dadurch gekennzeichnet, daß die eingesetzten Polymerisate ionogene und/oder nichtionische Monomere enthalten.

14. Verfahren nach einem der Ansprüche 2 bis 13, dadurch gekennzeichnet, daß die eingesetzten Polymerisate wenigstens teilweise als Ammonium-, Alkali- oder Erdalkalisalze vorliegen.

**15.** Verwendung der Granulate nach Ansprüche 1 bis 14 als Absorptionsmittel, insbesondere zur Aufnahme von Körperflüssigkeiten wie Urin, Blut oder zur Aufnahme und Speicherung von wässrigen Lösungen in Papier und textilen Materialien.

**16.** Wegwerferzeugnisse für Hygieneartikel, wie z.B. Windeln oder Damenbinden, enthaltend als Absorptionsmittel für Körperflüssigkeiten, wie Wasser und Urin ein Granulat nach Anspruch 1.

**Claims**

**1.** Granular material of water-swellable polymers characterized by a content of water-swellable polymer of 75 to 99.5, preferably of 80 to 98%-wt., and 25 to 0.5, preferably 20 to 2%-wt. of a powdery agglomeration auxiliary agent which causes the agglomeration by mere temperature elevation, thus effecting the melting or softening, and optional further additional non-agglomerating components, obtainable by heating a water-swellable, powdery, finely dispersed polymer having a particle size of 10 to 150, preferably 10 to 100 $\mu$m with the powdery agglomeration auxiliary agent in the absence of free water.

**2.** A process for the manufacture of granular material of water-swellable polymers according to claim 1 by sinter-agglomeration of powdery, finely dispersed starting materials, characterized in that a mixture comprising 75 to 99.5, preferably 80 to 98%-wt. of the powdery, water-swellable polymer and 25 to 0.5, preferably 20 to 2%-wt. of a powdery agglomeration auxiliary agent which, owing to its meltability or capability of softening, has an agglomerating effect caused by temperature elevation, is heated in the absence of free water.

**3.** The process according to claim 2 characterized in that a water-swellable polymer having a particle size of 10 to 150, preferably 10 to 100 $\mu$m is used as starting material.

**4.** The process according to any one of claims 2 to 3 characterized in that a mixture is heated that comprises 75 to 99.5, preferably 80 to 98%-wt. of water-swellable polymer and 25 to 0.5, preferably 20 to 2%-wt. of agglomeration auxiliary agent.

**5.** The process according to any one of claims 2 to 4 characterized in that substances which are powdery in the range of -100 to +300°C, preferably in the range of 20 to 200°C, and in particular in the range of 50 to 160°C are used as agglomeration auxiliary agents.

**6.** The process according to any one of claims 2 to 5 characterized in that synthetic or natural substances alone or in admixture with each other are used as agglomeration auxiliary agents, said substances having a melting/softening/point/range.

**7.** The process according to claim 6 characterized in that thermoplastic and/or thermosetting materials are used as agglomeration auxiliaries.

**8.** The process according to any one of claims 2 to 7 characterized in that polymers based on polyolefins, polyamides, polyesters, poly(meth-)acrylates, poly(meth-)acrylonitriles, polyalkylene oxides, polyvinyl chloride, polystyrene, polycarbonates, and/or polyurethanes are used as synthetic agglomeration auxiliary agents.

**9.** The process according to any one of claims 2 to 6 characterized in that epoxy resins, aminoplastics, phenolic plastics alone or in combination with a hardener are used as synthetic agglomeration auxiliaries.

**10.** The process according to any one of claims 2 to 6 characterized in that cellulose esters, shellac, colophony and its derivatives, or natural thermoplastics based on fatty acids and fatty acid derivatives are used as natural agglomeration auxiliary agents.

**11.** The process according to any one of claims 2-10 characterized in that the auxiliary substances used for the agglomeration additionally comprise non-agglomerating compounds, such as fillers, softeners, flow improving agents, separating agents, hardeners, antistatic agents, stabilizers, and/or foaming agents.

EP 0 321 755 B1

12. The process according to any one of clams 2 to 11 characterized in that polymers of (meth-)acrylic acid, polymers of at least partially saponified (meth-)acrylonitrile, polymers of at least partially saponified (meth-)acrylamide, polymers of at least partially saponified (meth-)acrylic-acid esters, graft copolymers of (meth-) acrylonitrile or (meth-)-acrylamide with starch or cellulose or their derivatives, polyurethanes, and/or polymers of isobutylene and maleic anhydride are used as water-swellable polymers.

13. The process according to any one of claims 2 to 12 characterized in that the polymers used comprise ionogenic and/or non-ionic monomers.

14. The process according to any one of claims 2 to 13 characterized in that the polymers used are present at least partially as salts of ammonium, alkali or alkaline earth.

15. The use of the granular material according to claims 1 to 14 as absorbents, in particular for the absorption of body liquids, such as urine, blood, or for the absorption and storage of aqueous solutions in paper and textile materials.

16. Disposable products for hygienic articles, such as diapers and sanitary napkins, comprising as absorbent for body liquids, such as water and urine, a granular material according to claim 1.

**Revendications**

1. Granulés de polymères gonflables à l'eau, qui se caractérisent par le fait de contenir 75 à 99,5% en poids, de préférence 80 à 98% en poids, de polymère gonflable à l'eau et de 25 à 0,5% en poids, de préférence 20 à 2% en poids, d'un adjuvant d'agglomération pulvérulent qui provoque l'agglomération sous le seul effet d'une élévation de la température et amorce, de ce fait, la fusion ou le ramollissement, ainsi qu'éventuellement des constituants supplémentaires, sans effet agglomérant, que l'on peut obtenir par le chauffage d'un polymère gonflable à l'eau, pulvérulent, finement dispersé, d'un calibre des particules de 10 à 150 $\mu$m, de préférence 10 à 100 $\mu$m, en même temps que de l'adjuvant d'agglomération pulvérulent, en l'absence d'eau libre.

2. Procédé de préparation de granulés de polymères gonflables à l'eau suivant la revendication 1, par une agglomération par frittage de matières de départ pulvérulentes et finement dispersées, caractérisé en ce que l'on chauffe, en l'absence d'eau libre, un mélange qui contient 75 à 99,5% en poids, de préférence 80 à 98% en poids, du polymère pulvérulent, gonflable à l'eau et 25 à 0,5% en poids, de préférence 20 à 2% en poids, d'un adjuvant d'agglomération pulvérulent, qui exerce une activité agglomérante en raison de sa fusibilité ou de son caractère ramollisable par élévation de la température.

3. Procédé suivant la revendication 2, caractérisé en ce que, à titre de matière de départ, on utilise un polymère gonflable à l'eau d'un calibre des particules de 10 à 150 $\mu$m, de préférence 10 à 100 $\mu$m.

4. Procédé suivant l'une quelconque des revendications 2 et 3, caractérisé en ce que l'on chauffe un mélange qui contient 75 à 99,5% en poids, de préférence 80 à 98% en poids, de polymère gonflable à l'eau et 25 à 0,5% en poids, de préférence 20 à 2% en poids, d'un adjuvant d'agglomération.

5. Procédé suivant l'une quelconque des revendications 2 à 4, caractérisé en ce que, à titre d'adjuvant d'agglomération, on utilise des substances qui sont pulvérulentes dans la plage de -100 à +300°C, de préférence dans la plage de 20 à 200°C et, plus particulièrement, dans la plage de 50 à 160°C.

6. Procédé suivant l'une quelconque des revendications 2 à 5, caractérisé en ce que, à titre d'adjuvant d'agglomération, on utilise des substances synthétiques ou naturelles, seules ou en mélange les unes aux autres, qui présentent une plage de points de fusion/ramollissement.

7. Procédé suivant la revendication 8, caractérisé en ce que l'on utilise des matières thermoplastiques et/ou duroplastiques à titre d'adjuvant d'agglomération.

11

**8.** Procédé suivant l'une quelconque des revendications 2 à 7, caractérisé en ce que l'on met en oeuvre, à titre d'adjuvants d'agglomération synthétiques, des polymères à base de polyoléfines, de polyamides, de polyesters, de poly(méth)acrylates, de poly(méth)acrylonitriles, de poly(oxydes d'alkylène), de poly(chlorure de vinyle), de polystyrène, de polycarbonates et/ou de polyuréthannes.

**9.** Procédé suivant l'une quelconque des revendications 2 à 6, caractérisé en ce que l'on utilise, à titre d'adjuvants d'agglomération synthétiques, des résines époxy, des matières aminoplastiques, des matières phénoplastiques, seules ou en commun avec un durcisseur.

**10.** Procédé suivant l'une quelconque des revendications 2 à 6, caractérisé en ce que, à titre d'adjuvants d'agglomération naturels, on utilise des esters de cellulose, le shellac, la colophane et ses dérivés, ou des matières thermoplastiques naturelles à base d'acides gras et de dérivés d'acides gras.

**11.** Procédé suivant l'une quelconque des revendications 2 à 10, caractérisé en ce que les adjuvants d'agglomération contiennent complémentairement des composés sans activité agglomérante, comme des charges, des plastifiants, des agents d'amélioration de l'écoulement ou de la fluidité, des agents de séparation, des durcisseurs, des composés antistatiques, des stabilisateurs et/ou des moussants.

**12.** Procédé suivant l'une quelconque des revendications 2 à 11, caractérisé en ce que l'on utilise, à titre de polymères gonflables à l'eau, des polymères de l'acide méth(acrylique), des polymères du (méth)acrylonitrile au moins partiellement saponifiés, des polymères du (méth)acrylamide au moins partiellement saponifiés, des polymères d'esters de l'acide (méth)acrylique au moins partiellement saponifiés, des copolymères de greffage du (méth)acrylonitrile ou du (méth)acrylamide avec l'amidon ou la cellulose ou leurs dérivés, des polyuréthannes et/ou des polymères de l'isobutylène et de l'anhydride maléique.

**13.** Procédé suivant l'une quelconque des revendications 2 à 12, caractérisé en ce que les polymères utilisés contiennent des monomères ionogènes et/ou non ioniques.

**14.** Procédé suivant l'une quelconque des revendications 2 à 13, caractérisé en ce que les polymères employés se présentent au moins partiellement sous forme des sels d'ammonium, de métaux alcalins ou de métaux alcalino-terreux.

**15.** Utilisation des granulés suivant l'une quelconque des revendications 1 à 14 à titre d'agents d'absorption, plus spécialement pour l'absorption de liquides corporels, comme l'urine, le sang, ou pour l'absorption et la rétention de solutions aqueuses dans le papier et les matières textiles.

**16.** Produits à jeter après l'emploi pour des articles hygiéniques, par exemple des couches ou des bandes cataméniales, contenant un granulé suivant la revendication 1, à titre d'agents d'absorption de liquides corporels, comme l'eau et l'urine.